# EUROPÄISCHE PATENTSCHRIFT

(11) **EP 2 345 400 B1**
(45) Veröffentlichungstag und Bekanntmachung des Hinweises auf die Patenterteilung: **24.10.2018**
(21) Anmeldenummer: 10189931.8
(22) Anmeldetag: 04.11.2010
(51) Int. Cl.: A61K 8/23, A61K 8/25, A61K 8/37, A61K 8/86, A61Q 5/10

(54) **Blondierungen mit verringerter Haarschädigung**
Lightening compositions having reduced hair damage
Compositions éclaircissantes abimant moins les cheveux

(30) Priorität: 16.12.2009 DE 102009054763
(43) Veröffentlichungstag der Anmeldung: 20.07.2011
(73) Patentinhaber: Henkel AG & Co. KGaA, 40589 Düsseldorf (DE)
(72) Erfinder: Janßen, Frank, 41470, Neuss (DE); Wadle, Armin, 40699, Erkrath (DE); Günther, Katja, 40721, Hilden (DE); Krippahl, Marc, 41239 Mönchengladbach (DE); Schmahl, Melanie, 40721, Hilden (DE)

(56) Entgegenhaltungen:
- EP-A1- 1 878 469
- EP-A2- 1 321 128
- EP-A2- 1 752 138
- DE-A1-102004 045 253
- FR-A1- 2 925 317
- US-A1- 2006 251 594
- US-A1- 2008 220 031

## Beschreibung

Gegenstand der vorliegenden Anmeldung sind Mehrkomponentenverpackungseinheiten und daraus hergestellte, anwendungsbereite Mittel zur Aufhellung oder Blondierung keratinischer Fasern, insbesondere menschlicher Haare. Die anwendungsbereiten Mittel zeichnen sich dabei durch den Gehalt von bestimmten nichtionischen Tensiden sowie Carbonsäureesterölen aus. Dadurch können effiziente Aufhellergebnisse mit reduzierter Haarschädigung und verringerter Kopfhautirritation erreicht werden.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Die Grundlagen der Blondierverfahren sind dem Fachmann bekannt und in einschlägigen Monographien beschrieben.

Zum Blondieren menschlicher Haare werden üblicherweise feste oder pastenförmige Zubereitungen mit festen Oxidationsmitteln unmittelbar vor der Anwendung mit einer verdünnten Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Aufhellung liegt zwischen etwa 30 und 60 Minuten. Es ist naheliegend, dass bei den Benutzern dieser Blondiermittel ein Bedürfnis besteht, diese Einwirkzeit zu verringern. Um eine ausreichenden Blondierwirkung zu erzielen, sind derartige Mittel üblicherweise stark alkalisch eingestellt, der pH-Wert liegt dabei zwischen 9 und 11,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen.

Gleichzeitig jedoch führen der Einsatz von Oxidationsmitteln und der alkalische pH-Wert zu Schädigungen in der Haarstruktur und auf der Haaroberfläche. Das Haar wird brüchig, seine Elastizität lässt nach und die Kämmbarkeit nimmt ab. Die Spreizung der äußeren Schuppenschicht führt außerdem zu einem unangenehmen Oberflächenempfinden der Haare und damit zu einer verschlechterten Kämmbarkeit im Nass- und Trockenzustand. Weiterhin kann es durch Alkalisierungsmittel und Oxidationsmittel zu Irritationen und Reizungen der Kopfhaut, insbesondere bei empfindlichen Anwendern, kommen. Dadurch besteht für den Verbraucher eine gesteigerte Notwendigkeit, zusätzliche konditionierende oder pflegende Nachbehandlungsmittel einsetzen.

Unter den aggressiven Bedingungen (starke Oxidationsmittel, hoher pH-Wert) der aufhellenden Zubereitung sowie der den im Stand der Technik üblichen Cremezubereitungen (meist pH > 11) sind die eingesetzten Pflegemittel zumeist nicht ausreichend stabil. Daher müssen Blondierschritt und Konditioniermittel üblicherweise nacheinander erfolgen. Aus Gründen des Anwendungskomforts ist aber eine einstufige Blondierung, die bereits entsprechende Pflegemittel beinhaltet, besonders vorteilhaft. Daher besteht ein Bedürfnis nach Blondiermitteln, die durch Zusatz von stabilen Pflegekomponenten in die Anwendungszubereitung zu einer verringerten Haarschädigung und reduzierten Kopfhautirritation führen.
Aufgabe der vorliegenden Erfindung ist es daher, die oben genannten Nachteile oxidativer Aufhell- und/oder Blondiermittel für menschliche Haare herabzusenken. Die Aufhellmittel sollen das Haar schützen und damit eine verringerte Schädigung des Haares bewirken. Insbesondere soll durch die Mittel dem Haar erhöhte Elastizität und Geschmeidigkeit sowie einen verbesserten Glanz verliehen werden. Weiterhin sollen Kopfhautirritationen vermindert werden. Die Verringerung von Haarschädigungen während der Aufhellung soll jedoch nicht zu Lasten einer verringerten Aufhellleistung der Mittel erreicht werden.
Es wurde nun in nicht vorhersehbarer Weise gefunden, dass der Zusatz von bestimmten nichtionischen Tensiden sowie Carbonsäureesterölen in Aufhellmitteln für keratinische Fasern zu Vorteilen gegenüber herkömmlichen Aufhellmitteln hinsichtlich Glanz und Pflege der Fasern sowie Reduktionen von Kopfhautirritationen führt.
Ein erster Gegenstand der Erfindung ist daher eine Mehrkomponentenverpackungseinheit (Kit-of-Parts), welche mindestens drei getrennt voneinander konfektionierte Container nach Anspruch 1 umfasst, worin ein erster Container (I) mindestens eine Oxidationszubereitung (A), enthaltend in einem flüssigen, kosmetischen Träger als Oxidationsmittel mindestens Wasserstoffperoxid sowie als nichtionisches Tensid mindestens einen ethoxylierten Fettalkohol mit 10 bis 30 Ethylenoxideinheiten, enthält, ein zweiter Container (II) mindestens ein Blondierpulver (B), enthaltend als Bleichkraftverstärker mindestens ein Persulfatsalz, ausgewählt aus der Gruppe, gebildet aus Kaliumperoxodisulfat, Natriumperoxodisulfat und Ammoniumperoxodisulfat, und zusätzlich Siliciumdioxid, enthält, wobei die Mehrkomponentenverpackungseinheit dadurch gekennzeichnet ist, dass entweder die Oxidationszubereitung (A) des ersten Containers (I) oder ein weiterer getrennt konfektionierter Container (III) oder (IV) zusätzlich mindestens ein Carbonsäureesteröl mit einem Gesamtgehalt an 12 bis 80 C-Atomen, ausgewählt aus der Gruppe, die gebildet wird aus
i. Monoestern aus je einem Äquivalent Carbonsäure und Alkanol,
enthält.
Ein kosmetischer flüssiger Träger ist im Sinne der Erfindung wässrig, alkoholisch oder wässrigalkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haaraufhellung und/oder Blondierung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

In einer besonders bevorzugten Ausführungsform ist die Verpackungseinheit des ersten Erfindungsgegenstands dadurch gekennzeichnet, dass sie mindestens eine zusätzliche Komponente, ausgewählt aus der Gruppe, die gebildet wird aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Unter einem Container ist im Rahmen der Erfindung ein Behältnis in Form einer Dose, eines Fläschchens, einer Tüte oder eines Sachets zu verstehen. Dabei können unterschiedliche Materialien, insbesondere Kunststoffe, eingesetzt werden. Die Container können dabei, wo Bedarf danach besteht, wiederverschließbar sein.

Die Oxidationszubereitung (A) des ersten Containers (I) enthält als Oxidationsmittel Wasserstoffperoxid. Wasserstoffperoxid kann dabei als wässrige Lösung oder in Form eines seiner Anlagerungsprodukte an organische oder anorganische Verbindungen, enthalten. Bevorzugte Anlagerungsprodukte sind die Anlagerungsprodukte von Wasserstoffperoxid an Harnstoff, Melamin sowie Natriumborat. Bevorzugt wird jedoch als Oxidationsmittel Wasserstoffperoxid in Form einer wässrigen Lösung eingesetzt. Bevorzugt beträgt die Menge an Wasserstoffperoxid in der Oxidationsmittelzubereitung (A) 3 bis 25 Gew.-%, bevorzugt 6 bis 20 Gew.-% (berechnet als 100 %-iges H₂O₂), jeweils bezogen auf das Gesamtgewicht der Oxidationsmittelzubereitung (A).

Als weiteren wesentlichen Inhaltsstoff enthält die Oxidationszubereitung (A) des ersten Containers (I) als nichtionisches Tensid mindestens einen ethoxylierten Fettalkohol mit 10 bis 30 Ethylenoxideinheiten. Erfindungsgemäß ist darunter ein Anlagerungsprodukt von Ethylenoxid an einen Fettalkohol zu verstehen. Fettalkohole sind dabei gesättigte und ungesättigte Alkohole mit 12 bis 24 C-Atomen, welche linear oder verzweigt sein können. Die Molmenge an Ethylenoxid, die pro Mol Fettalkohol eingesetzt wurde, bezeichnet dabei den Ethoxylierungsgrad verstanden. Als nichtionisches Tensid eignen sich dabei insbesondere Ethylenoxid-Anlagerungsprodukte an Octylalkohol (Caprylalkohol), Nonylalkohol (Pelargonylalkohol), Undecylalkohol, Undec-10-en-1-ol, Dodecylalkohol (Laurylalkohol), 2,6,8-Trimethyl-4-nonanol (Isolaurylalkohol), Tridecylalkohol, Tetradecylalkohol (Myristylalkohol), Pentadecylalkohol, Hexadecylalkohol (Cetyl-/ Palmitylalkohol), Heptadecylalkohol, Octadecylalkohol (Stearylalkohol), Isostearylalkohol, (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenylalkohol), Nonadecan-1-ol (Nonadecylalkohol), Eicosan-1-ol (Eicosylalkohol / Arachylalkohol), (9Z)-Eicos-9-en-1-ol (Gadoleylalkohol), (5Z,8Z,11Z,14Z)-Eicosa-5,8,11,14-tetraen-1-ol (Arachidonalkohol), Heneicosylalkohol, Docosylalkohol (Behenylalkohol), (13Z)-Docos-13-en-1-ol (Erucylalkohol) oder (13E)-Docosen-1-ol (Brassidylalkohol). Es ist erfindungsgemäß ebenfalls möglich, Gemische von Fettalkoholen, die durch gezielte Mischung oder auch durch Gewinnungsverfahren als solche anfallen, einzusetzen. Beispiele sind Cocosalkohol (Mischung aus C₈-C₁₈-Fettalkoholen) oder Cetearylalkohol (1:1-Mischung aus C₁₆- und C₁₈-Fettalkoholen).

Erfindungsgemäß bevorzugte nichtionisches Tenside vom Typ ethoxylierter Fettalkohol sind insbesondere Laureth-10, Laureth-12, Laureth-15, Laureth-20, Laureth-30, Myreth-10, Myreth-12, Myreth-15, Myreth-20, Myreth-30, Ceteth-10, Ceteth-12, Ceteth-15, Ceteth-20, Ceteth-30, Steareth-10, Steareth-12 Steareth-15, Steareth-20, Steareth-30, Oleth-10, Oleth-12, Oleth-15, Oleth-20, Oleth-30, Ceteareth-10, Ceteareth-15, Ceteareth-12, Ceteareth-15, Ceteareth-20, Ceteareth-30 sowie Coceth-10, Coceth-12, Coceth-15, Coceth-20 und Cocoeth-30. Bevorzugt sind insbesondere Ethoxylierungsgrade von 15 bis 25.

Eine weitere Ausführungsform des ersten Erfindungsgegenstands ist dadurch gekennzeichnet, dass als nichtionisches Tensid ein ethoxylierter Fettalkohol mit 15 bis 25 Ethylenoxideinheiten enthalten ist.

Insbesondere bevorzugte ethoxylierte Fettalkohole sind Laureth-20, Steareth-20, Steareth-16, Steareth-25, Ceteth-20, Ceteth-16, Ceteth-15, Ceteth-24, Ceteth-25, Ceteareth-20, Ceteareth-23, Ceteareth-15, Ceteareth-25, Oleth-20, Oleth-15 und Oleth-16, besonders bevorzugt Ceteareth-20.

Bevorzugt beträgt der Anteil der ethoxylierten Fettalkohole mit 10 bis 30 Ethylenoxideinheiten in der Oxidationsmittelzubereitung (A) 0,1 bis 5 Gew.-% und bevorzugt 0,5 bis 3 Gew.-%, jeweils bezogen auf das Gesamtgewicht der Oxidationsmittelzubereitung (A).

Das Blondierpulver (B) des zweiten Containers (II) enthält als Bleichkraftverstärker mindestens ein Persulfatsalz, ausgewählt aus der Gruppe, gebildet aus Kaliumperoxodisulfat, Natriumperoxodisulfat und Ammoniumperoxodisulfat. Weiterhin hat es sich bei den Arbeiten zur vorliegenden Erfindung als besonders bevorzugt erwiesen, wenn das Blondierpulver mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Die Peroxodisulfatsalze sind in einer Menge von 20 bis 99 Gew.-% bezogen auf das Gesamtgewicht des Blondierpulvers (B), enthalten.

Zur weiteren Steigerung der Aufhellleistung können der erfindungsgemäßen Zusammensetzung mindestens eine gegebenenfalls hydratisierte SiO₂-Verbindung zugesetzt. Dadurch kann die Aufhellleistung signifikant gesteigert werden. Bevorzugte gegebenenfalls hydratisierte SiO₂-Verbindungen sind Kieselsäuren, deren Oligomeren und Polymeren sowie deren Salze, insbesondere Natriumsalze, und Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt. Auch Metasilicate, die sich gemäß vorstehender Formel durch das Verhältnis zwischen n und der Summe aus m und p von ≤ 1 auszeichnen und sich als kettenförmige polymere Strukturen des Anions [SiO₃]²⁻ auffassen lassen, können bevorzugt eingesetzt werden. Natriummetasilicat der Formel [NaSiO₃]ₓ, ist dabei besonders bevorzugt. Besonders bevorzugte Blondierpulver enthalten jedoch zusätzlich mindestens Siliciumdioxid (Kieselsäure; Silicagel).

Es hat sich erfindungsgemäß als bevorzugt erwiesen, wenn das Blondierpulver (B) wasserfrei formuliert ist. Wasserfrei im Sinne der vorliegenden Erfindung bedeutet einen Wassergehalt bezogen auf das Pulver (B) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Das Blondierpulver (B) ist weiterhin bevorzugt staubfrei formuliert.

Die Blondierpulver (B) enthalten weiterhin bevorzugt zusätzlich ein festes Alkalisierungsmittel. Verwendbare Alkalisierungsmittel werden gewählt aus anorganischen Salzen, insbesondere der Alkali- und Erdalkalimetalle, und festen, organischen Alkalisierungsmitteln, insbesondere basischen Aminosäuren. Es hat sich aber im Rahmen der Untersuchungen zur vorliegenden Erfindung herausgestellt, dass erfindungsgemäß bevorzugte Blondierpulver (B) dadurch gekennzeichnet sind, dass sie ein festes, anorganisches Alkalisierungsmittel enthalten. Das erfindungsgemäße, anorganische Alkalisierungsmittel wird bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie deren Mischungen.
Das Blondierpulver (B) enthält die festen Alkalisierungsmittel bevorzugt in Mengen von 0,2 bis 25 Gew.-%, insbesondere 0,5 bis 10 Gew.-%, bezogen auf das Gesamtgewicht des Blondierpulvers (B).

Als weiteren wesentlichen Inhaltsstoff enthält die Mehrkomponentenverpackungseinheit des Erfindungsgegenstands mindestens ein Carbonsäureesteröl mit einem Gesamtgehalt an 12 bis 80 C-Atomen, die ausgewählt sind aus der Gruppe, die gebildet wird aus
i. Monoestern aus je einem Äquivalent Carbonsäure und Alkanol.

Die Esteröle stellen dabei zumindest formal Reaktionsprodukte aus Carbonsäure und Alkohol dar, die unter Abspaltung von Wasser eine Carbonsäureesterfunktionalität bilden. Dabei kommen als Carbonsäuren sowie als Alkanole sowohl gesättigte und ungesättigte als auch lineare oder verzweigte Verbindungen in Frage, solange die Kohlenstoffgesamtzahl zwischen 12 und 80 liegt. Die Esteröle sind bevorzugt bei Raumtemperatur flüssige Verbindungen. Je nach Herkunft kann es sich bei den Alkanolen und Carbonsäuren jeweils um Isomerengemische, wie beispielsweise Isotridecylalkohole, oder auch um Gemische aus verschiedenen Verbindungen mit unterschiedlicher Kohlenstoffanzahl, wie beispielsweise natürliche oder herstellungsbedingte Fettsäureschnitte oder Fettalkoholschnitte, handeln.
Erfindungsgemäße Beispiele für Esteröle vom Typ der Monoester aus je einem Äquivalent Carbonsäure und Alkanol sind Cetyl Lactat, Lauryl Lactat, C₁₂-C₁₅-Alkyl Lactat, Isostearyl Lactat, Linoleyl Lactat, Oleyl Lactat, Isocetyl Caprylat, Capryl Caprylat, Cetyl Caprylat, Decyl Caprylat, Isodecyl Caprylat, Isostearyl Caprylat, Stearyl Caprylat, Propylheptyl Caprylat (Caprinsäure-2-propylheptylester), Decyl Oleat, Isodecyl Oleat, Cetyl Oleat, Cetyl Ethylhexanoat (2-Ethylhexansäurecetylester), Ethylhexyl Olivat (2-Ethylhexylester des Fettsäurenschnitts aus Olivenöl), Ethylhexyl Isononanoat (3,5,5-Trimethylhexansäure-2-ethylhexylester), Capryl Isononanoat, Cetearyl Isononanoat, Isononyl Isononanoat, Isotridecyl Isononoanat, Octyldodecyl Behenat (Behensäure-2-octyldodecylester), Isocetyl Behenat, Capryl Pelargonat, Tridecyl Erucat, Octyldodecyl Erucat (Erucasäure-2-octyldodecylester), Oleyl Erucat, Ethyl Palmitat, Isopropyl Palmitat, Ethylhexyl Palmitat (Palmitinsäure-2-ethylhexylester), Octyldodecyl Palmitat, Butyl Palmitat, Cetyl Palmitat, Myristyl Palmitat, Stearyl Palmitat, Capryl Palmitat, Isostearyl Palmitat, Ethyl Myristat, Isopropyl Myristat, Ethylhexyl Myristat (Myristinsäure-2-ethylhexylester), Octyldodecyl Myristat, Butyl Myristat, Cetyl Myristat, Myristyl Myristat, Stearyl Myristat, Capryl Myristat, Isostearyl Myristat, Hexyl Stearat, Ethylhexyl Stearat, Butyl Stearat, Isobutyl Stearat, Capryl Stearat, Isocetyl Stearat, Myristyl Stearat, Isocetyl Isostearat, Hexyl Laurat, Hexyldecyl Laurat (Laurinsäure-2-hexyldecylester), Isocetyl Laurat, Isopropyl Laurat und Cocosfettalkohol-caprinat/-caprylat (Estergemisch aus Caprinsäure- /Caprylsäureestern mit Cocosfettalkoholen).

Bevorzugt sind dabei Carbonsäureesteröle, welche 12 bis 40 C-Atome, insbesondere 12 bis 25 C-Atomen enthalten. Bevorzugte Carbonsäureesteröle sind dabei Propylheptyl Caprylat, Lauryl Lactat, Cetyl Ethylhexanoat, Isotridecyl Isononanoat, Isopropyl Palmitat sowie Isopropyl Myristat.

In der Ausführungsform des Erfindungsgegenstands enthält die Mehrkomponentenverpackungseinheit mindestens einen weiteren Container (III), welcher eine Alkalisierungszubereitung (C) enthält. Eine solche Alkalisierungszubereitung (C) enthält in einem flüssigen, kosmetischen Träger mindestens ein Alkalisierungsmittel, wobei bevorzugte Alkalisierungsmittel ausgewählt sind aus Ammoniak und Alkanolaminen, insbesondere Monoethanolamin und 2-Amino-2-methylethanol. Das Carbonsäureesteröl ist entweder getrennt konfektioniert oder in die Formulierungen der Oxidationszubereitung (A) oder der Alkalisierungszubereitung (C) eingearbeitet.

Der Erfindungsgegenstand ist daher eine Mehrkomponentenverpackungseinheit (Kit-of-Parts), die dadurch gekennzeichnet ist, dass das Kit-of-Parts mindestens einen dritten getrennt konfektionierten Container umfasst, wobei der dritte Container (III) mindestens eine Alkalisierungszubereitung (C), enthaltend in einem flüssigen, kosmetischen Träger mindestens ein Alkalisierungsmittel, ausgewählt aus Ammoniak, Monoethanolamin und/oder 2-Amino-2-methylpropanol, enthält, und
dass entweder die Oxidationszubereitung (A) des ersten Containers (I) oder die Alkalisierungszubereitung (C) des dritten Containers (III) oder ein vierter getrennt konfektionierter Container (IV) zusätzlich mindestens ein Carbonsäureesteröl mit einem Gesamtgehalt an 12 bis 80 C-Atomen, ausgewählt aus der Gruppe, die gebildet wird aus
i. Monoestern aus je einem Äquivalent Carbonsäure und Alkanol,
enthält.
Die Komponenten der jeweiligen Container einer Mehrkomponentenverpackungseinheit werden zu einem anwendungsbereiten Mittel zum Aufhellen von keratinischen Fasern, insbesondere menschlichen Haaren vermischt.
Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Dabei enthält die Mehrkomponentenverpackungseinheit die einzelnen Zubereitungen in aufeinander abgestimmten Gewichtsverhältnissen. Bevorzugt werden auf 10 Gewichtsteile einer Oxidationszubereitung (A) 1 bis 6 Gewichtsteile des Blondierpulvers (B), gegebenenfalls 1 bis 2 Gewichtsteile der Ölkomponente aus Container (IV) sowie gegebenenfalls 8 bis 10 Gewichtsteile der Alkalisierungszubereitung (C) eingesetzt.

Um eine vorzeitige, unerwünschte Reaktion der Inhaltsstoffe untereinander zu verhindern, werden die Inhaltstoffe zweckmäßigerweise getrennt voneinander konfektioniert und erst unmittelbar vor der Anwendung in Kontakt gebracht.

Ein zweiter Gegenstand der vorliegenden Erfindung ist daher ein anwendungsbereites Mittel zur Aufhellung menschlicher Haare, welches dadurch gekennzeichnet ist, dass es aus den Komponenten einer Mehrkomponentenverpackungseinheit des ersten Erfindungsgegenstands durch Vermischen der einzelnen Zubereitungen unmittelbar vor der Anwendung auf dem Haar hergestellt wird.

Bevorzugt beträgt die Menge an Wasserstoffperoxid als Oxidationsmittel im anwendungsbereiten Mittel 1 bis 12 Gew.-%, bevorzugt 2 bis 10 Gew.-% insbesondere bevorzugt zu 3 bis 8 Gew.-% (berechnet als 100 %-iges H₂O₂), jeweils bezogen auf das anwendungsbereite Mittel.

Eine weitere Ausführungsform des zweiten Erfindungsgegenstands ist dadurch gekennzeichnet, dass das Mittel ein oder mehrere Carbonsäureesteröle in einem Anteil von 0,1 Gew.-% bis 30 Gew.-%, bevorzugt 1,0 Gew.-% bis 20 Gew.-% und besonders bevorzugt von 2,0 Gew.-% bis 15 Gew.-%, jeweils bezogen auf das Gesamtgewicht des anwendungsbereiten Mittels, enthält.

Eine weitere Ausführungsform des zweiten Erfindungsgegenstands ist dadurch gekennzeichnet, dass das Mittel einen oder mehrere ethoxylierte Fettalkohole mit 10 bis 30 Ethylenoxideinheiten in einem Anteil von 0,1 Gew.-% bis 3 Gew.-%, bevorzugt 0,2 Gew.-% bis 2,0 Gew.-% und besonders bevorzugt von 0,3 Gew.-% bis 1,5 Gew.-%, jeweils bezogen auf das Gesamtgewicht des anwendungsbereiten Mittels, enthält.

Weiterhin hat es sich als vorteilhaft erweisen, wenn die Aufhellmittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Zur Steigerung der Aufhellwirkung ist ebenso möglich, als zusätzliche Bleichkraftverstärker kationisierte Heterocyclen zuzugeben. Geeignete Bleichkraftverstärker vom Typ kationisierter Heterocyclen sind insbesondere physiologisch verträgliche Salze von Acetyl-1-methylpyridinium, 4-Acetyl-1-methylpyridinium und N-Methyl-3,4-dihydroisochinolinium, besonders bevorzugt 2-Acetyl-1-methylpyridinium-p-toluolsulfonat, 4-Acetyl-1-methylpyridinium-p-toluolsulfonat und N-Methyl-3,4-dihydroisochinolinium-p-toluolsulfonat.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen, bevorzugt 8 bis 24 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen), polyethoxylierte Ethercarbonsäuren, Acylsarcoside, Acyltauride, Acylisethionate, Sulfobernsteinsäuremono- und -dialkylester und Sulfobernsteinsäuremono-alkylpolyoxyethylester mit 1 bis 6 Oxyethylgruppen, lineare Alkansulfonate, lineare α-Olefinsulfonate, Sulfonate ungesättigter Fettsäuren mit bis zu 6 Doppelbindungen, α-Sulfofettsäuremethylester von Fettsäuren, Alkylsulfate und Alkylethersulfate mit zu bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, sowie Monoglyceridsulfate und Monoglyceridethersulfate.

Bevorzugte anionische Tenside sind Seifen, Alkylsulfate, Alkylethersulfate und Ethercarbonsäuren mit 10 bis 18 C-Atomen in Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

In einer weiteren Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer C₈-C₂₄-Alkyl- oder -Acylgruppe im Molekül mindestens eine freie Aminogruppe und mindestens eine COOH- oder SO₃H-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate mit den Handelsnamen Miranol C2M SF conc., Amphoterge K-2 und Monateric CEM-38 und Bezeichnung Disodium Cocoamphodiacetate mit den Handelsnamen Dehyton, Miranol C2M und Ampholak XCO 30 vermarktet.

Weiterhin hat es sich als vorteilhaft erwiesen, wenn die erfindungsgemäßen Aufhellmittel zusätzliche nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise zusätzliche Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; mit einem Methyl- oder C₂-C₆-Alkylrest-end-gruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe; C₁₂-C₃₀-Fettsäuremono- und -diester von Anlagerungsprodukten von 1 bis 30 Mol Ethylenoxid an Glycerin; Polyglycerinester und alkoxylierte Polyglycerinester, wie Poly(3)glycerindiisostearat und Poly(2)-glycerinpolyhydroxy-stearat; Anlagerungsprodukte von 5 bis 60 Mol Ethylenoxid an Rizinusöl und gehärtetes Rizinusöl; Polyolfettsäureester; ethoxylierte Mono-, Di- und Triglyceride; alkoxylierte Fettsäurealkylester; Aminoxide; Hydroxymischether; Sorbitanfettsäureester und Anlagerungsprodukte von Ethylenoxid an Sorbitanfettsäureester wie Polysorbate; Zuckerfettsäureester und Anlagerungsprodukte von Ethylenoxid an Zuckerfettsäureester; Anlagerungsprodukte von Ethylenoxid an Fettsäurealkanolamide und Fettamine; Fettsäure-N-alkylglucamide sowie Alkylpolyglykoside.

Als nichtionische Tenside eignen sich Alkylpolyglykoside, insbesondere C₈-C₂₂-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 14 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Die anionischen, nichtionischen, amphoteren oder zwitterionischen Tenside werden in Gesamtmengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Stearamidopropyldimethylamin dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben.

Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die erfindungsgemäßen Mittel können weiterhin mindestens einen direktziehenden Farbstoff enthalten. Prinzipiell sind der Wahl der direktziehenden Farbstoffe keine Grenzen gesetzt. Erfindungsgemäß einsetzbare direktziehende Farbstoffe sind Nitrophenylendiamine, Nitroaminophenole, Azofarbstoffe, Anthrachinone oder Indophenole. Direktziehende Farbstoffe werden üblicherweise in anionische, kationische und nichtionische direktziehende Farbstoffe unterteilt. Es ist jedoch erfindungsgemäß besonders vorteilhaft, wenn die direktziehenden Farbstoffe eine hinreichende Stabilität gegenüber den harschen Bedingungen des Blondierprozesses besitzen.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)-amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bevorzugt enthält das Mittel als direktziehenden Farbstoff mindestens einen anionischen direktziehenden Farbstoff, ausgewählt aus Bromphenolblau, Tetrabromphenolblau und/oder aus der Gruppe der Fluorescin-Farbstoffe Acid Red 92 (auch D&C RED No. 28 oder Phloxin B), Acid Red 98, Acid Red 94, Acid Red 87, oder Acid Red 51, und/oder mindestens einen neutralen, direktziehenden Farbstoff, ausgewählt aus 4-[(2,3-Dihydroxypropyl)amino]-3-nitro-1-trifluormethyl-benzol (HC Yellow 6), 1-Chlor-2,4-bis[(2-hydroxyethyl)amino]-5-nitrobenzol (HC Yellow 10), 1-Chlor-4-[(2-hydroxyethyl)amino]-3-nitrobenzol (HC Yellow 12) und 4-[(2-Hydroxyethyl)amino]-3-nitro-1-trifluormethyl-benzol (HC Yellow 13).

Erfindungsgemäß bevorzugte Farbstoffkombinationen sind solche, die mindestens die Kombination aus Tetrabromphenolblau und Acid Red 92; Tetrabromphenolblau und Acid Red 98; Tetrabromphenolblau und Acid Red 94; Tetrabromphenolblau und Acid Red 87; oder Tetrabromphenolblau und Acid Red 51; enthalten. Erfindungsgemäß besonders bevorzugt sind Mittel, die mindestens eine Farbstoffkombination aus Tetrabromphenolblau und Acid Red 92 enthalten.

Es ist erfindungsgemäß besonders vorteilhaft, wenn im anwendungsbereiten Mittel die Gesamtmenge an direktziehenden Farbstoffen 0,0001 bis 2,5 Gew.-%, bezogen auf das Gesamtgewicht des anwendungsbereiten Mittels, beträgt.

Ferner können die erfindungsgemäßen Mittel weitere Wirk-, Hilfs- und Zusatzstoffe enthalten, wie beispielsweise Assoziativpolymere mit Fettalkylkette, kationische Polymere, nichtionische Polymere (Vinylpyrrolidinon/Vinylacrylat-Copolymere, Polyvinylpyrrolidinon, Vinylpyrrolidinon/ Vinylacetat-Copolymere, Polyethylenglykole und Polysiloxane); zwitterionische und amphotere Polymere (Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butyl-acrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (Phospholipide, wie Sojalecithin, Ei-Lecitin, Kephaline sowie Silikonöle); Proteinhydrolysate pflanzlicher oder tierischer Herkunft (Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, B₃, B₅, B₆, C, E, F und H; Pflanzenextrakte (aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Moringa, Hauhechel, Huflattich, Eibisch, Meristem, Ginseng und Ingwer); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol / Acrylamid-Copolymere); Perlglanzmittel (Ethylenglykolmono- und -distearat sowie PEG-3-distearat); Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Blondierzubereitungen weisen bevorzugt einen pH-Wert im Bereich von 7 bis 12, bevorzugt einen pH-Wert im Bereich von 8,0 bis 11,5 auf. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-1-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat, bevorzugt Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Schließlich ist ein weiteres bevorzugtes Alkalisierungsmittel Ammoniak.

Bezüglich weiterer bevorzugter Ausführungsformen der Mittel des zweiten Erfindungsgegenstands gilt mutatis mutandis das zu den erfindungsgemäßen Mehrkomponentenverpackungseinheiten (Kit-of-Parts) Gesagte.

Ein weiterer Gegenstand der vorliegenden Erfindung ist zur Aufhellung und/oder Blondierung menschlicher Haare, wobei ein Mittel des zweiten Erfindungsgegenstands auf das Haar aufgetragen wird, für einen Zeitraum von 3 bis 45 Minuten, bevorzugt 5 bis 30 Minuten im Haar belassen wird, und das Haar anschließend mit Wasser und/oder einem handelsüblichen Shampoo gespült wird. Die Auftragungs- und die Einwirktemperatur der Blondierzubereitung beträgt Raumtemperatur bis 45°C. Gegebenenfalls kann die Wirkung der Blondierzubereitung durch externe Wärmezufuhr, wie beispielsweise mittels einer Wärmehaube, verstärkt werden. Die bevorzugte Einwirkdauer der Blondierzubereitung auf die keratinische Faser beträgt 3 bis 60 min, bevorzugt 5 bis 45 min. Nach Beendigung der Einwirkdauer wird das verbliebene Blondiermittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet. Die Auftragung der Blondierzubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Blondierzubereitung mit einer Applikationshilfe auf die keratinischen Fasern aufzutragen.

Ein weiterer Gegenstand der vorliegenden Erfindung ist die Verwendung eines Mittels des zweiten Erfindungsgegenstands zur Verringerung der Haarschädigung bei der oxidativen Aufhellung menschlicher Haare.

Ein weiterer Gegenstand der vorliegenden Erfindung ist die Verwendung eines Mittels des zweiten Erfindungsgegenstands zur Verringerung der Kopfhautirritation bei der oxidativen Aufhellung menschlicher Haare.

Bezüglich weiterer bevorzugter Ausführungsformen der erfindungsgemäßen Verfahren und Verwendungen gilt mutatis mutandis das zu den erfindungsgemäßen Mitteln Gesagte.

### Beispiele

Die Mengenangaben in den Beispielen verstehen sich, soweit nichts anderes vermerkt ist, in Gewichtsprozent. Es wurden die folgenden Zubereitungen hergestellt:

### 1 Oxidationsmittelzubereitungen (A) (in Gew.-%)

| **Rohstoff** | **A1** | **A2** | **A3** |
|---|---|---|---|
| Natriumbenzoat | 0,04 | 0,05 | 0,04 |
| Dipicolinsäure | 0,10 | 0,15 | 0,10 |
| Dinatriumpyrophosphat | -- | 0,15 | 0,10 |
| Dinatriumphosphat | 0,10 | -- | -- |
| 1,2-Propandiol | -- | 1,80 | 1,50 |
| Kaliumhydroxid 50% | 0,12 | 0,22 | 0,19 |
| HEDP, 60% | 0,25 | 0,30 | 0,25 |
| Isopropyl Myristat | 0,80 | -- | -- |
| Dibutyl Adipat | -- | -- | 5,00 |
| Cetearylalkohol | 4,00 | 4,00 | 3,40 |
| Ceteareth-20 | 1,00 | 1,20 | 1,00 |
| Bienenwachs | 0,30 | -- | -- |
| Wasserstoffperoxid, 50 % | 24,00 | 21,60 | 18,20 |
| Wasser | ad 100 | ad 100 | ad 100 |

### 2 Blondierpulver (B) (in Gew.-%)

| **Rohstoff** | **B1** | **B2** |
|---|---|---|
| Kaliumperoxodisulfat | 98,6 | -- |
| Natriumperoxodisulfat | -- | 14,5 |
| Ammoniumperoxodisulfat (incl. 0.5% Silica) | -- | 10,0 |
| Silica, hydrophil | 1,4 | 0,4 |
| Natriummetasilicat | -- | 11,8 |
| Portil N | -- | 23,8 |
| Natriumhexametaphosphat | -- | 0,2 |
| Rohagit S hv | -- | 2,0 |
| EDTA, Na₂-Salz | -- | 0,6 |
| Celquat L200 | -- | 0,3 |
| Cekol 50000 | -- | 1,0 |
| Paraffinum Liquidum | -- | 9,7 |
| Magensiumcarbonat | -- | ad 100 |

### 3 Alkalisierungsmittel (C) (in Gew.-%)

| **Rohstoff** | **C1** | **C2** |
|---|---|---|
| Decyl Oleat | 1,7 | 1,4 |
| Lanette N | 10,2 | 8,5 |
| Cetearylalkohol | 2,9 | 2,4 |
| Cutina GMS SE | 4,3 | 3,6 |
| Cocosamidopropylbetain, 40% | 1,4 | 1,2 |
| Monoethanolamin | 9,6 | 8,0 |
| Dibutyl Adipat | -- | 10,0 |
| Wasser, entsalzt | ad 100 | |

Rohstoffe: Lanette N (INCI-Bezeichnung: Cetearyl Alcohol, Sodium Cetearyl Sulfate; Cognis); Cutina GMS SE (INCI-Bezeichnung: Glyceryl Stearate; Cognis); Portil N (INCI-Bezeichnung: Sodium Silicate; Cognis); Rohagit S hv (INCI-Bezeichnung: Acrylates Copolymer; Evonik); Celquat L200 (INCI-Bezeichnung: Polyquaternium-4; National Starch); Cekol 50000 (INCI-Bezeichnung: Cellulose Gum; CP Kelco).

### 4 Mehrkomponentenverpackungseinheiten

Folgende Mehrkomponentenverpackungseinheiten wurden zusammengestellt (Zubereitung 4.2 ist erfindungsgemäß und Zubereitungen 4.1, 4.3 und 4.4 nicht):

| | | | | |
|---|---|---|---|---|
| 4.1 | 4K-Mittel | Container (I): | Oxidationsmittelzubereitung A1 | 50 g |
| | | Container (II): | Blondierpulver B1 | 10 g |
| | | Container (III): | Alkalisierungsmittel C1 | 50 g |
| | | Container (IV): | Dibutyl Adipat | 10 g |
| 4.2 | 3K-Mittel | Container (I): | Oxidationsmittelzubereitung A1 | 50 g |
| | | Container (II): | Blondierpulver B1 | 10 g |
| | | Container (III): | Alkalisierungsmittel C2 | 50 g |
| 4.3 | 3K-Mittel | Container (I): | Oxidationsmittelzubereitung A2 | 50 g |
| | | Container (II): | Blondierpulver B2 | 30 g |
| | | Container (III): | Dibutyl Adipat | 10 g |
| 4.4 | 2K-Mittel | Container (I): | Oxidationsmittelzubereitung A3 | 50 g |
| | | Container (II): | Blondierpulver B2 | 25 g |

Unmittelbar vor der Anwendung auf den Fasern wurden die einzelnen Zubereitungen jeweils innig miteinander vermischt. Die resultierenden Anwendungszubereitungen wurden jeweils auf Strähnen von dunkelbraunem Haar in einem Flottenverhältnis von 4 zu 1 aufgetragen und 45 min bei 35 °C auf den Strähnen belassen. Anschließend wurden die Strähnen mit Wasser gründlich gespült und mit Hilfe eines Föns getrocknet.

## Patentansprüche

1. Mehrkomponentenverpackungseinheit (Kit-of-Parts), umfassend mindestens drei getrennt voneinander konfektionierte Container, worin
ein erster Container (I) mindestens eine Oxidationszubereitung (A), enthaltend in einem flüssigen, kosmetischen Träger als Oxidationsmittel mindestens Wasserstoffperoxid sowie als nichtionisches Tensid mindestens einen ethoxylierten Fettalkohol mit 10 bis 30 Ethylenoxideinheiten, enthält,
ein zweiter Container (II) mindestens ein Blondierpulver (B), enthaltend als Bleichkraftverstärker mindestens ein Persulfatsalz, ausgewählt aus der Gruppe, gebildet aus Kaliumperoxodisulfat, Natriumperoxodisulfat und Ammoniumperoxodisulfat, und zusätzlich eine gegebenenfalls hydratisierte SiO₂-Verbindung, enthält,
ein dritter Container (III) mindestens eine Alkalisierungszubereitung (C), enthaltend in einem flüssigen, kosmetischen Träger mindestens ein Alkalisierungsmittel, ausgewählt aus Ammoniak, Monoethanolamin und/oder 2-Amino-2-methylpropanol, enthält,
**dadurch gekennzeichnet,**
**dass** entweder die Oxidationszubereitung (A) des ersten Containers (I) oder die Alkalisierungszubereitung (C) des dritten Containers (III) oder ein vierter getrennt konfektionierter Container (IV) zusätzlich mindestens ein Carbonsäureesteröl mit einem Gesamtgehalt an 12 bis 80 C-Atomen, ausgewählt aus der Gruppe, die gebildet wird aus
i. Monoestern aus je einem Äquivalent Carbonsäure und Alkanol,
enthält.

2. Mehrkomponentenverpackungseinheit nach Anspruch 1, **dadurch gekennzeichnet, dass** als Carbonsäureesteröl ein Carbonsäureesteröl mit 12 bis 25 C-Atomen enthalten ist.

3. Mehrkomponentenverpackungseinheit nach einem der Ansprüche 1 bis 2, **dadurch gekennzeichnet, dass** als nichtionisches Tensid ein ethoxylierter Fettalkohol mit 15 bis 25 Ethylenoxideinheiten enthalten ist.

4. Anwendungsbereites Mittel zur Aufhellung menschlicher Haare, **dadurch gekennzeichnet, dass** es aus den Komponenten einer Mehrkomponentenverpackungseinheit nach einem der Ansprüche 1 bis 3 durch Vermischen der einzelnen Zubereitungen unmittelbar vor der Anwendung auf dem Haar hergestellt wird.

5. Mittel nach Anspruch 4, **dadurch gekennzeichnet, dass** das Mittel ein oder mehrere Carbonsäureesteröle in einem Anteil von 0,1 Gew.-% bis 30 Gew.-%, bevorzugt 1,0 Gew.-% bis 20 Gew.-% und besonders bevorzugt von 2,0 Gew.-% bis 15 Gew.-%, jeweils bezogen auf das Gesamtgewicht des anwendungsbereiten Mittels, enthält.

6. Mittel nach einem der Ansprüche 4 oder 5, **dadurch gekennzeichnet, dass** das Mittel einen oder mehrere ethoxylierte Fettalkohole mit 10 bis 30 Ethylenoxideinheiten in einem Anteil von 0,1 Gew.-% bis 3 Gew.-%, bevorzugt 0,2 Gew.-% bis 2,0 Gew.-% und besonders bevorzugt von 0,3 Gew.-% bis 1,5 Gew.-%, jeweils bezogen auf das Gesamtgewicht des anwendungsbereiten Mittels, enthält.

7. Verwendung eines Mittels nach einem der Ansprüche 4 bis 6 zur Verringerung der Haarschädigung bei der oxidativen Aufhellung menschlicher Haare.

## Claims

1. A kit of parts, comprising at least three separately packaged containers, in which a first container (I) contains at least one oxidizing preparation (A), containing, in a liquid cosmetic carrier, at least hydrogen peroxide as an oxidizing agent and at least one ethoxylated fatty alcohol having 10 to 30 ethylene oxide units as a non-ionic surfactant, a second container (II) contains at least one blonding powder (B), containing, as a bleach enhancer, at least one persulfate salt selected from the group formed of potassium peroxodisulfate, sodium peroxodisulfate and ammonia peroxodisulfate, and additionally an optionally hydrated SiO₂ compound, a third container (III) contains at least one alkalizing preparation (C), containing, in a liquid cosmetic carrier, at least one alkalizing agent selected from ammonia, monoethanolamine and/or 2-amino-2-methlypropanol, **characterized in that** either the oxidizing preparation (A) of the first container (I) or the alkalizing preparation (C) of the third container (III) or a fourth separately packaged container (IV) also contains at least one carboxylic acid ester oil having a total content of 12 to 80 C atoms, selected from the group formed of
i. monoesters made of one carboxylic acid equivalent and one alkanol equivalent.

2. The kit of parts according to claim 1, **characterized in that** a carboxylic acid ester oil having 12 to 25 C atoms is contained as the carboxylic acid ester oil.

3. The kit of parts according to one of claims 1 to 2, **characterized in that** an ethoxylated fatty alcohol having 15 to 25 ethylene oxide units is contained as the non-ionic surfactant.

4. A ready-to-use agent for lightening human hair, **characterized in that** it is prepared from the components of a kit of parts according to one of claims 1 to 3 by mixing the individual preparations immediately before application to the hair.

5. The agent according to claim 4, **characterized in that** the agent contains one or more carboxylic acid ester oils in a proportion of 0.1 wt.% to 30 wt.%, preferably 1.0 wt.% to 20 wt.%, particularly preferably of 2.0 wt.% to 15 wt.%, based in each case on the total weight of the ready-to-use agent.

6. The agent according to one of claims 4 or 5, **characterized in that** the agent contains one or more ethoxylated fatty alcohols having 10 to 30 ethylene oxide units in a proportion of 0.1 wt.% to 3 wt.%, preferably 0.2 wt.% to 2.0 wt.% and particularly preferably of 0.3 wt.% to 1.5 wt.%, based in each case on the total weight of the ready-to-use agent.

7. The use of an agent according to one of claims 4 to 6 for reducing damage to hair when oxidatively lightening human hair.

## Revendications

1. Conditionnement de plusieurs composants (ensemble de composants) comprenant au moins trois contenants confectionnés de manière distincte dont un premier contenant (1) contient au moins une préparation oxydante (A), comprenant au moins du peroxyde d'hydrogène, comme oxydant, ainsi qu'un alcool gras éthoxylé de 10 à 30 unités d'oxyde d'éthylène, comme tensioactif non ionique, dans une substance porteuse cosmétique fluide,
un deuxième contenant (II) contient au moins une poudre décolorante (B), comprenant au moins un sel persulfate, comme amplificateur de décoloration, choisi dans le groupe qui se forme de persulfate de potassium, de persulfate de sodium et de persulfate d'ammonium, et en plus d'une éventuelle liaison hydratée de SiO₂, un troisième contenant (III) contient au moins une préparation alcalinisante (C), comprenant au moins un agent alcalinisant, choisi entre l'ammoniaque, la monoéthanolamine et/ou le 2-amino-2-méthylpropanol, dans une substance porteuse cosmétique fluide, **caractérisé en ce que** ni la préparation oxydante (A) du premier contenant (I), ni la préparation alcalinisante (C) du troisième contenant (III), ni un quatrième contenant (IV) confectionné de manière distincte ne contient en plus une huile à base d'ester d'acide carboxylique avec une teneur totale de 12 à 80 atomes de carbone, choisie dans le groupe qui se forme de
i. monoesters composés chacun d'un équivalent d'acide carboxylique et d'alcanol.

2. Conditionnement de plusieurs composants, selon la revendication 1, **caractérisé en ce qu'**une huile à base d'ester d'acide carboxylique comprenant 12 à 25 atomes de carbone est contenue comme huile à base d'ester d'acide carboxylique.

3. Conditionnement de plusieurs composants, selon l'une des revendications 1 à 2, **caractérisé en ce qu'**un alcool gras éthoxylé comprenant 15 à 25 unités d'oxyde d'éthylène est contenu comme tensioactif non ionique.

4. Agent prêt à l'emploi permettant d'éclaircir les cheveux humains, **caractérisé par le fait qu'**il est fabriqué à partir des composants contenus dans un conditionnement de plusieurs composants, selon l'une des revendications 1 à 3, en mélangeant les différentes préparations juste avant l'application sur les cheveux.

5. Agent, selon la revendication 4, **caractérisé en ce que** ledit agent contient une ou plusieurs huiles à base d'ester d'acide carboxylique dans une proportion de 0,1 % du poids à 30 % du poids, de préférence de 1,0 % du poids à 20 % du poids, et encore plus préférablement de 2,0 % du poids à 15 % du poids, en se rapportant toujours au poids total de l'agent prêt à l'emploi.

6. Agent, selon l'une des revendications 4 ou 5, **caractérisé en ce que** ledit agent contient un ou plusieurs alcools gras éthoxylés comprenant entre 10 et 30 unités d'oxyde d'éthylène dans une proportion de 0,1 % du poids à 3 % du poids, de préférence de 0,2 % du poids à 2,0 % du poids, et encore plus préférablement de 0,3 % du poids à 1,5 % du poids, en se rapportant toujours au poids total de l'agent prêt à l'emploi.

7. Utilisation d'un agent, selon l'une des revendications 4 à 6, permettant de limiter les dommages que subissent les cheveux humains lorsqu'ils sont éclaircis par oxydation.
